(19) Europäisches Patentamt
European Patent Office
Office européen des brevets

(11) Numéro de publication: **0 017 543**
**B1**

(12) **FASCICULE DE BREVET EUROPEEN**

(45) Date de publication du fascicule du brevet: **28.09.83**

(21) Numéro de dépôt: **80400368.9**

(22) Date de dépôt: **19.03.80**

(51) Int. Cl.[3]: C 07 D 277/66, A 61 K 31/425 //C07D277/82

(54) **Dérivés du benzothiazole, leur procédé de préparation et leurs applications en thérapeutique.**

(30) Priorité: **03.04.79 FR 7908303**

(43) Date de publication de la demande:
**15.10.80 Bulletin 80/21**

(45) Mention de la délivrance du brevet:
**28.09.83 Bulletin 83/39**

(84) Etats contractants désignés:
**AT CH DE GB LU NL SE**

(56) Documents cités:
**FR - A - 2 106 462**
**FR - A - 2 198 757**

(73) Titulaire: **Albert ROLLAND S.A. Société dite
49, Rue St-André-des-Arts
F-75006 Paris (FR)**

(72) Inventeur: **Lepant, Marcel
Résidence Lescoundu Chemin Claire Fontaine
F-06140 Vence (FR)**
Inventeur: **Bessin, Pierre
1 Allée Bossuet
F-91380 Chilly Mazarin (FR)**
Inventeur: **Bonnet, Jacqueline
19 Rue Charcot
F-75013 Paris (FR)**

(74) Mandataire: **Bressand, Georges et al,
c/o CABINET LAVOIX 2 Place d'Estienne d'Orves
F-75441 Paris Cedex 09 (FR)**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 017 543 B1

Dérivés du benzothiazole, leur procédé de préparation et leurs applications en thérapeutique.

La présente invention concerne des dérivés de l'acide phényl-2 benzothiazolyl-6, acétique.

La Demande de brevet FR—A 2 106 462 décrit les acides phényl-2 benzothiazolyl-5 et 6 acétiques et les propriétés anti-inflammatoires, analgésiques et antipyrétiques de ces composés.

La demande de brevet FR—A 2 198 757 et l'article du Journal of Medicinal Chemistry, 1974 vol. 17 No. 5 pages 491—496 décrivent l'acide (phényl-2-benzothiazolyl-5)-2-propionique et ses propriétés anti-inflammatoires et analgésiques.

La Demanderesse a trouvé que l'acide (phényl-2 benzothiazolyl-6)-2 propionique présentant des propriétiés pharmacologiques inattendues par rapport à celles de ces composés.

La présente invention a donc pour objet les deux stéréoisomères et le racémique de l'acide (phényl-2 benzothiazolyl-6)-2 propionique de formule

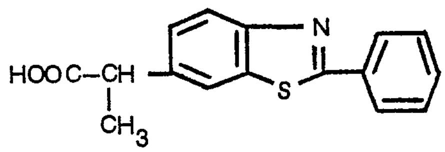

(I)

et leurs sels avec les bases physiologiquement acceptables, cations alcalins, alcalino-terreux et amines, ainsi que leurs sels avec les acides physiologiquement acceptables, minéraux ou organiques, et leurs esters d'alkyle.

Les acides racémiques de formule I peuvent être préparés par réaction sur l'aminothiol de formule II ou un équivalent tel le disulfure correspondant à la formule III

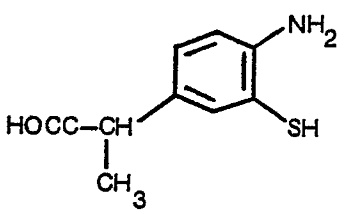

(II)

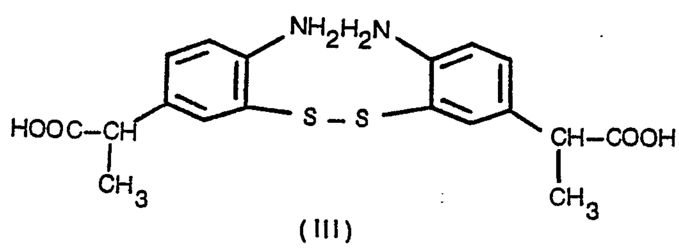

(III)

de l'acide benzoïque ou d'un dérivé tel que le chlorure de benzoyle, un ester de benzoyle ou l'aldéhyde benzoïque, la réaction avec le chlorure de benzoyle conduisant au meiller rendement.

Lors de 1 action de chlorure d'acide sur l'aminothiol de formule II, qui a lieu de préférence en présence d'une base minérale ou organique, il se forme en particulier les composés intermédiaires IV et V

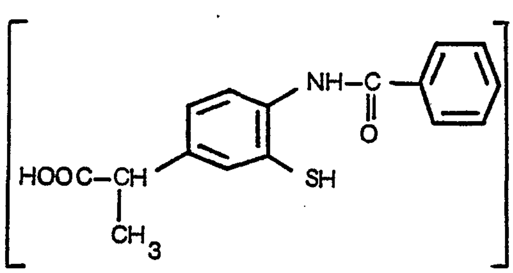

(IV)

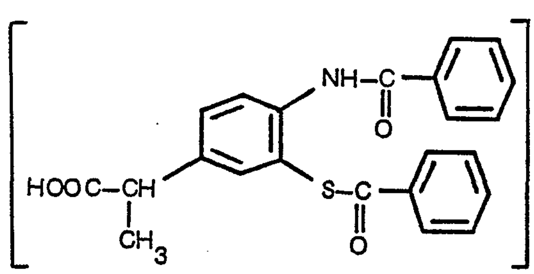

(V)

Ces composés de formules IV et V ne sont pas purifiés mais leur mélange est cyclisé soit par l'action de la chaleur, soit par action des acides ou des bases.

Lors de l'action de la benzaldéhyde sur l'aminothiol de formule II, qui a lieu soit dans un solvant indifférent, tel que nitrobenzène, benzène, . . . soit dans un solvant basique, tel que pyridine, quinoléine, il peut se former intermédiairement une benzothiazine de formule VI

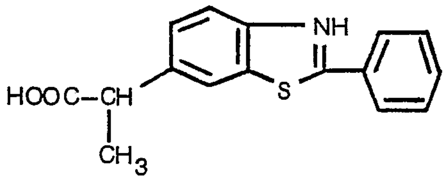

(VI)

qui par oxydation, par exemple par $FeCl_3$ ou le diméthylsulfoxyde, donnera le benzothiazole recherché.

Les deux énantiomères de l'acide de formule I peuvent être séparés en utilisant un sel de l'acide correspondant avec une amine chirale, et plus particulièrement chacun des deux énantiomères de la phényl-2 éthylamine. Selon une méthode connue en soi, on recristallise ces sels pour séparer les deux diastéréo-isomères.

Les sels des acides de formule I peuvent être préparés par action d'une quantité stoechiométrique de l'acide ou de la base choisie sur les acides de formule I en solution dans un alcool, une cétone ou un éther aliphatique.

Les esters des acides de formule I peuvent étre préparés par action d'un alcool ou d'un halogénoalkane sur les acides de formule I ou l'un de leurs dérivés réactifs, selon des méthodes connues en soi.

Les aminothiols de formule II peuvent être préparés par décomposition, en milieu basique, en présence ou non d'un réducteur, d'un composé de formule

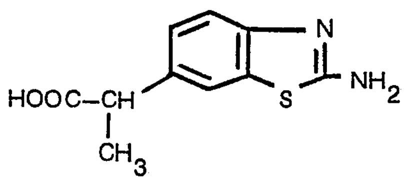

(VII)

Ces aminoacides sont des produits chimiques nouveaux, qui peuvent être préparés par action du brome et du thiocyanate d'ammonium, sur un composé de formule

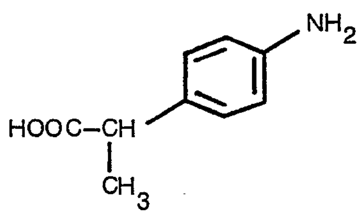

(VIII)

en solution dans l'acide acétique.

Les exemples suivants illustrent la préparation des composés de la formule (I).

EXEMPLE 1

a) Préparation de l'acide (amino-2 benzothiazolyl-6)-2 propionique

On dissout 24,7 g d'acide (amino-4, phényl)-2 propionique, préparé par exemple comme dans Org. Prep. Proc. Int. p. 303—307 (1977), dans 300 ml d'acide acétique, puis 22,8 g de thiocyanate d'ammonium. A 15°C, on introduit, goutte à goutte dans cette solution 24 g de brome en solution dans 60 ml d'acide acétique, puis laisse le mélange revenir à température ambiante sous agitation, pour quelques heures. on élimine alors le solvant sous pression réduite et met l'huile rédiduelle en suspension dans une solution aqueuse acide; on élimine le précipité et amène la phase aqueuse à pH 4,5 par addition d'une solution d'hydroxyde de sodium. Le précipité formé est isolé par filtration et recristallisé dans le méthanol ou l'éthanol pour donner 26 g d'acide (amino-2 benzothiazolyl-6)-2 propionique pur de point de fusion 234°C.

b) Préparation de l'acide (phényl-2 benzothiazolyl-6)-2 propionique en utilisant le chlorure de benzoyle

On porte à la température du reflux jusqu'à fin de dégagement d'ammoniac une solution de 45 g d'acide (amino-2 benzothiazolyl-6)-2 propionique dans 200 ml de solution aqueuse d'hydroxyde de sodium à 40% contenant ou non 40 g de sulfure de sodium, nonahydrate. Environ 500 ml d'eau sont introduits dans la solution et à la température de 10°C, on ajoute lentement sous forte agitation à cette solution aqueuse, 70 g de chlorure de benzoyle en solution à 10% dans un solvant non miscible à l'eau, tel le benzène. Après la fin de l'addition, on laisse le mélange réactionnel revenir à température ambiante et continue l'agitation quelques heures avant de séparer la phase organique et d'acidifier la

phase aqueuse jusqu'à pH 4 par addition d'acide chlorhydrique en solution aqueuse concentrée. Le précipité apparu contient de l'acide benzoïque, les dérivés non cyclisés, de type IV et V, et parfois même du benzothiazole final.

La cyclisation est effectuée sur ce mélange par exemple par chauffage du solide gommeux à 120°C plusieurs heures, ou encore en milieu acide comme suit: le solide est dissous dans 1 litre d'eau contenant 70 ml d'une solution aqueuse d'hydroxyde de sodium à 40%; la solution est filtrée sur noir décolarant, puis versée, goutte à goutte, dans une solution aqueuse environ 2 N d'acide chlorhydrique, maintenue vers 50°C; le précipité apparu est isolé par filtration à cette température.

L'acide final, dont 45 g sont ainsi obtenus, est recristallisé dans l'isopropanol aqueux (2/1) ou dans un mélange butanone-2/cyclohexane (1/2) pour donner 36 g de produit pur fondant vers 160°C. Suivant les solvants de recristallisation et la vitesse de précipitation, l'acide se présentera sous des formes cristallines différentes, de points de fusion parfois très proches mais dont les spectres infrarouge en phase solide ne sont pas superposables.

La cyclisation peut aussi être effectuée en milieu basique: on dissout alors le solide obtenu abondammet lavé à l'eau, dans 350 ml d'une solution d'éthanol aqueux à 95% contenant 25 g d'hydroxyde de potassium, puis après quelques heures de chauffage, on acidifie, élimine l'éthanol et verse dans 1 litre d'eau environ, Le précipité d acide final qui se forme est isolé et recristallisé comme précédemment mentionné.

EXEMPLE 2

Préparation de l'acide (phényl-2 benzothiazolyl-6)-2 propionique en utilisant la benzaldéhyde

22 g d'acide (amino-2 benzothiazolyl-6)-2 propionique sont dissous dans 100 ml d'une solution concentrée d'hydroxyde de sodium et le mélange porté 1 heure à sa température de reflux (fin de dégagement d'ammoniac). A température ambiante, on amène la solution à pH 4,5 par addition d'une solution d'acide chlorhydrique et filtre le précipité apparu. On isole ainsi 16 g de précipité de point de fusion voisin de 154°C. Ce solide et 8 g de benzaldéhyde sont dissous dans 100 ml de pyridine et le mélange maintenu à sa température de reflux 5 heures. On élimine alors le solvant sous pression réduite. Le résidu est dissous dans 100 ml d'éthanol, 200 mg de chlorure ferrique sont ajoutés et la solution est maintenue 2 heures à sa température de reflux. Le précipité apparu au refroidissement est purifié par dissolution dans une solution aqueuse basique avant d'être recristallisé dans un mélange butanone-2/cyclohexane (1/2). On obtient le produit final pur avec 40% de rendement.

EXEMPLE 3

Le sel de sodium de l'acide (phényl-2 benzothiazolyl-6(-2 propionique hygroscopique est préparé par action de 0,55 g d'hydroxyde de sodium sur 3,7 g d'acide en solution dans 100 ml de méthanol. F = 270°C. Ce sel est soluble dans l'eau.

EXEMPLE 4

Le sel de pyrrolidine de l'acide (phényl-2 benzothiazolyl-6)-2 propionique est préparé par action d'l g d'amine sur 4 g d'acide en solution dans le méthanol. Il fond à 142°C. Ce sel est soluble dans l'eau.

EXEMPLE 5

Le méthanesulfonate de l'acide (phényl-2 benzothiazolyl-6)-2 propionique est préparé par action de 0,1 mole d'acide sulfonique sur 0,1 mole d'acide en solution dans 500 ml d'acétone. F = 182°C.

EXEMPLE 6

Le chlorhydrate de l'acide (phényl-2 benzothiazolyl-6)-2 propionique est préparé par action d'acide chlorhydrique gazeux sur l'acide en solution dans le benzène. Il fond à 150°C en se décomposant.

EXEMPLE 7

Préparation du (phényl-2 benzothiazolyl-6)-2 propionate de méthyle

On dissout 6 g d'acide (phényl-2 benzothiazolyl-6)-2 propionique dans 100 ml de méthanol anhydre et porte au reflux du solvant 6 heures aprés addition de quelques gouttes d'acide sulfurique concentré. La solution est concentrée de moitié et l'ester précipité par addition d'un volume d'eau glacée. On obtient ainsi 5 g d'ester qui fond à 86°C.

EXEMPLE 8

Séparation des 2 énantiomères de l'acide (phényl-2 benzothiazolyl-6)-2 propionique à partir de leur mélange racémique.

a) Enantiomère dextrogyre (en solution dans le diméthylformamide)

On prépare le sel de l'acide avec l'$\alpha$-phényl éthylamine lévogyre dans la butanone-2. Par addition de 19 g d'amine en solution dans 50 ml de butanone-2 à une solution de 50 g d'acide dans 500 ml de butanone-2, il y a précipitation de 51 g de sel. Celui-ci est recristallisé six fois dans la quantité minimale

de butanone-2 pour donner 10 g de sel de pouvoir rotatoire spécifique, dans le méthanol (C = 2 g/100 ml) de : $(\alpha)_D^{22} = -22{,}5$°C.

L'acide est libéré par addition d'acide chlorhydrique à la solution aqueuse de ce sel d'amine. Le précipité isolé est recristallisé plusieurs fois dans des mélanges isopropanol/eau (2/1) pour donnere avec 35% de rendement l'énantiomère dextrogyre. F = 179°C.

b) Enantiomère lévogyre (en solution dans le diméthylformamide)

Les solvants de recristallisation du sel chiral obtenus selon (a) sont évaporés et l'acide, légèrement enrichi en énantiomère lévogyre, est libéré de son sel. On obtient ainsi 25 g d'acide dont on prépare 28 g de sel par action de 10,5 g d'$\alpha$-phényléthylamine dextrogyre sur l'acide en solution dans 540 ml de butanone-2.

Après trois recristallisations dans la butanone-2 le sel isolé a un pouvoir rotatoire: $(\alpha)_D^{22} = 23$° (C = 2 g/100 ml dans le méthanol).

L'acide est alors libéré de son sel et recristallisé plusieurs fois successivement dans des mélanges isopropanol/eau (2/1) et butanone-2/cyclohexane (1/2) pour donner avec 25% de rendement l'isomère lévogyre de pureté optique proche de 98%. $(\alpha)_D^{22} = -81$° dans le diméthylformamide (C = 0,1 g/ml) et de point de fusion 179°C.

Les composés de formule I et leurs dérivés sont des inhibiteurs de la biosynthèse des prostaglandines, et présentent des propiétés antalgiques aux doses inhibitrices. Ils possèdent également une activité antiagrégante plaquettaire, et protègent contre les effets cardiovasculaires du choc à l'endotoxine.

Leur toxicité est faible ($DL_{50}$ chez la souris par voie orale supérieure à 1000 mg/kg). Les doses efficaces mesurées dans les différents essais étant nettement plus faibles, l'indice thérapeutique de ces composés est excellent, ce qui permet leur utilisation en thérapeutique humaine.

L'activité analgésique des composés de formule I a été mise en évidence dans le test des torsions à la phénylbenzoquinone chez la souris. Ainsi, l'acide de formule I (racémique) possède dans ce test une $DE_{50}$ de 3 à 6 mg/kg P.O. Cette activité analgésique est prolongée dans le temps, une dose de 12,5 mg/kg de ce composé entraînant encore une analgésie de 42%, 24 heures après son administration.

Par ailleurs, les composés de formule I diminuent l'agrégation plaquettaire provoquée in vitro par l'ADP sur du plasma de rat enrichi en plaquettes; c'est ainsi que le (phényl-2 benzothiazolyl-6)-2 propionate de sodium est actif à une concentration dix fois plus faible que l'acide acétyl salicylique.

L'activité des composés de formule I sur la biosynthèse des prostaglandines se traduit par un effet protecteur contre la toxicité de l'acide arachidonique précurseur de cette biosynthèse. Ainsi, les composés de formule I administrés à la dose de 0,5 mg/kg par voie orale provoquent une diminution de la mortalité des souris ayant reçu une dose léthale d'acide arachidonique. Par voie intraveineuse, les sels solubles des composés de formule I, et notamment le sel de sodium, sont actifs aux mêmes doses. L'énantiomère dextrogyre de l'acide (exemple 8) a une activité comparable à celle du racémique (exemple 1), et supérieure à celle de son homologue lévogyre.

Enfin, les composés de formule I administrés par voie intraveineuse à la dose de 1 mg/kg, exercent un effet protecteur chez le chien anesthésié, contre les effets cardiovasculaires du choc à l'endotoxine d'Escherichia Coli.

Les composés de formule I peuvent être administrés sous différentes formes à l'homme, par voie orale, rectale ou parentérale, à des doses efficaces et non toxiques pour le traitement de la douleur, des hyperthermies, des dysménorrhées, des états de choc ou encore des maladies dans lesquelles la diminution de l'agrégation plaquettaire est un facteur important.

La dose unitaire dépend de la forme d'administration et du composé choisi comme principe actif du médicament. Les solutions pour administration parentérale ou intraveineuse sont préparées de préférence avec des diluants aqueux, et le principe actif étant sous une forme soluble dans ce milieu et par exemple sous forme d'un sel de cation alcalin. Pour l'administration orale, les composés de l'invention peuvent être introduits dans des gélules ou mélangés aux excipients habituels pour former des pilules, cachets ou comprimés libérant de façon immédiate les ingrédients actifs ou entérosolubles.

La posologie journalière chez l'adulte est, sauf cas particulier, comprise entre 20 mg et 1 g, en une ou plusieurs prises.

**Revendications pour les Etats Contractants: CH DE GB W NL SE**

1. Composé de formule

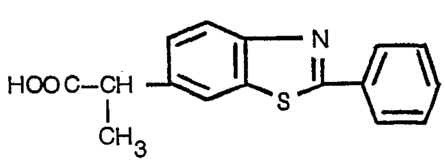

(I)

ses deux énantiomères et le racémique, leurs sels avec des bases physiologiquement acceptables, cations minéraux et amines, leurs sels avec les acides minéraux ou organiques physiologiquement acceptables et leurs esters d'alkyle.

2. Composition pharmaceutique, caractérisée en ce qu'elle comprend à titre de principe actif au moins l'un des composés selon la revendication 1.

3. Procédé de préparation des composés de formule I, caractérisé en ce que l'on fait réagir l'acide benzoïque ou un équivalent réactionnel, tel que chlorure d'acide, ester ou aldéhyde correspondants, sur un composé de formule

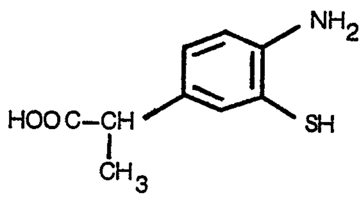

ou un équivalent de ce composé tel que le disulfure correspondant et éventuellement on convertit l'acide obtenu en un sel ou en un ester.

4. Procédé de séparation des deux énantiomères de l'acide (phényl-2 benzothiazolyl-6)-2 propionique caractérisé en ce que l'on effectue par recristallisation de sels diastéréoisomères préparés par action du racémique avec une amine chirale.

**Revendications pour l'Etat contractant: AT**

1. Procédé de préparation du composé de formule

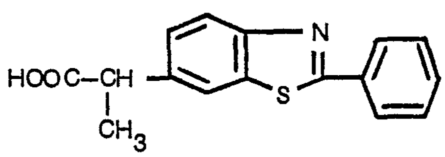

(I)

ses deux énantiomères et le racémique, leurs sels avec les bases physiologiquement acceptables, cations minéraux et amines, et leur sels avec les acides minéraux ou organiques physiologiquement acceptables et leurs esters d'alkyle, caractérisé en ce que l'on fait réagir l'acide benzoïque ou un équivalent réactionnel, tel que chlorure d'acide, ester ou aldéhyde correspondants, sur un composé de formule

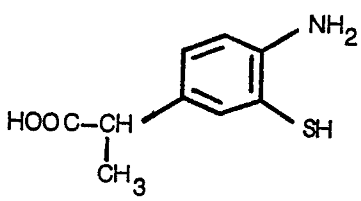

ou un équivalent de ce composé tel que le disulfure correspondant et éventuellement on convertit l'acide obtenu en un sel ou en un ester.

2. Procédé de séparation des deux énantiomères de l'acide (phényl-2 benzothiazolyl-6)-2 propionique caractérisé en ce qu'on l'effectue par recristallisation de sels diastéréoisomères préparés par action du racémique avec une amine chirale.

**Claims for the contracting states: CH DE GB W NL SE**

1. Compound having the formula

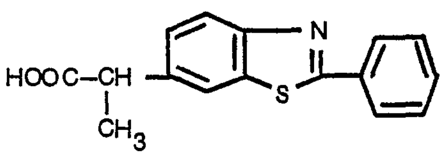

(I)

both its enantiomers and the racemate, their salts with physiologically acceptable bases, inorganic cations and amines, and their salts with physiologically acceptable inorganic or organic acids and their alkyl esters.

2. Therapeutic composition, characterized in that it comprises, as active ingredient, at least a compound as claimed in claim 1.

3. Process for the preparation of compounds of the formula (I), characterized in that benzoic acid or a reactive equivalent thereof, such as the corresponding acid chloride, ester or aldehyde is reacted, with a compound of the formula

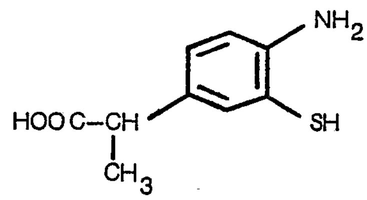

or an equivalent of said compound, such as the corresponding disulfide, and optionally converting the resulting acid to a salt or an ester.

4. Process for the separation of the two enantiomers of 2-(2-phenyl-6-benzothiazolyl) propionic acid, characterized in that the diastereoisomeric salts prepared by reaction of the racemate with a chiral amine are recrystallized.

**Claims for the contracting state: AT**

1. Process for the preparation of a compound having the formula

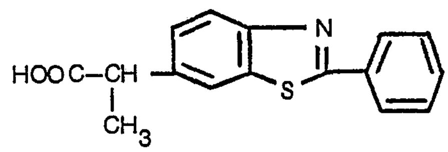

(I)

both its enantiomers and the racemate, their salts with physiologically acceptable bases, inorganic cations and amines, and their salts with physiologically acceptable inorganic or organic acids and their alkyl esters, characterized in that benzoic acid or a reactive equivalent thereof, such as the corresponding acid chloride, ester or aldehyde is reacted, with a compound of the formula

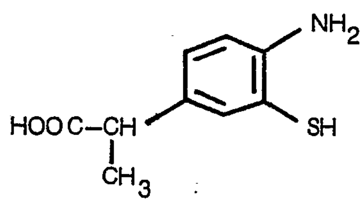

or an equivalent of said compound, such as the corresponding disulfide, and optionally converting the resulting acid to a salt or an ester.

2. Process for the separation of the two enantiomers of 2-(2-phenyl-6-benzothiazolyl) propionic acid, characterized in that the diastereoisomeric salts prepared by reaction of the racemate with a chiral amine are recrystallized.

**Patentansprüche für die Vertragsstaaten: CH DE GB W NL SE**

1. Verbindung der Formel

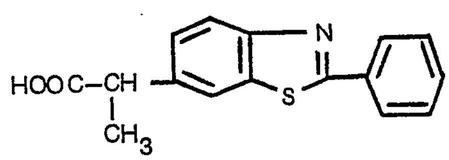

(I)

ihre beiden Enantiomeren und das Racemat, ihre Salze mit physiologisch annehmbaren Basen, anorganischen Kationen und Aminen, ihre Salze mit anorganischen oder organischen, physiologisch annehmbaren Säuren und ihre Alkylester.

2. Pharmazeutische Zubereitung, dadurch gekennzeichnet, daß sie als Wirkstoff wenigstens eine der Verbindungen gemäß Anspruch 1 enthält.

3. Verfahren zur Herstellung der Verbindungen der Formel I, dadurch gekennzeichnet, daß man die Benzoesäure oder ein reagierendes Äquivalent, z.B. das entprechende Säurechlorid, den entsprechenden Ester oder Aldehyd, mit einer Verbindung der Formel.

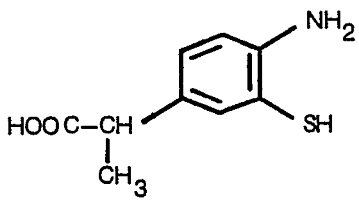

oder einem Äquivalent dieser Verbindung, z.B. dem entsprechenden Disulfid, umsetzt und die erhaltene Säure gegebenenfalls in ein Salz oder in einen Ester umwandelt.

4. Verfahren zur Trennung der beiden Enantiomeren der 2-(2-Phenyl-6-benzothiazolyl)-propionsäure, dadurch gekennzeichnet, daß man sie durch Umkristallisation der durch Einwirkung des Racemats auf ein chirales Amin hergestellten diastereomeren Salze bewirkt.

**Patentansprüche für den Vertragsstaat: AT**

1. Verfahren zur Herstellung der Verbindung der Formel

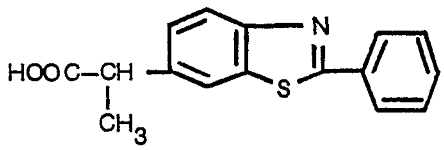

(I)

ihrer beiden Enantiomeren und des Racemats, ihrer Salze mit den physiologisch annehmbaren Basen, anorganischen Kationen und Aminen, und ihrer Salze mit anorganischen oder organischen, physiologisch annehmbaren Säuren und ihrer Alkylester, dadurch gekennzeichnet, daß man die Benzoesäure oder ein reagierendes Äquivalent, z.B das entsprechende Säurechlorid, den entsprechenden Ester oder Aldehyd, mit einer Verbindung der Formel

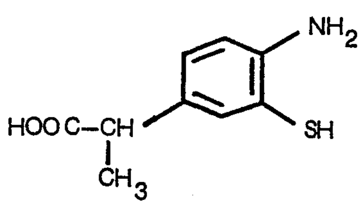

oder einem Äquivalent dieser Verbindung, z.B. dem entsprechenden Disulfid, umsetzt und die erhaltene Säure gegebenenfalls in ein Salz oder in einen Ester umwandelt.

2. Verfahren zur Trennung der beiden Enantiomeren der 2-(2-Phenyl-6-benzothiazolyl)-propionsäure, dadurch gekennzeichnet, daß man sie durch Umkristallisation der durch Einwirkung des Racemats auf ein chirales Amin hergestellten diastereomeren Salze bewirkt.